# EUROPEAN PATENT SPECIFICATION

(11) **EP 2 124 766 B1**
(45) Date of publication and mention of the grant of the patent: **26.06.2013**
(21) Application number: 08731780.6
(22) Date of filing: 10.03.2008
(51) Int. Cl.: A61B 17/22, A61B 17/221, A61B 17/00, A61B 18/14

(54) **TWO-STAGE SNARE-BASKET MEDICAL DEVICE**
ZWEISTUFIGER MEDIZINISCHER SCHLINGENFÄNGER
DISPOSITIF MÉDICAL D'ANSE À DEUX ÉTAGES

(30) Priority: 09.03.2007 US 894022 P; 31.10.2007 US 982245
(43) Date of publication of application: 02.12.2009
(73) Proprietor: Schwartz, Jeremy, New York, NY 10023 (US)
(72) Inventor: Schwartz, Jeremy, New York, NY 10023 (US)
(74) Representative: Cohausz & Florack
(86) International application number: PCT/US2008/056360
(87) International publication number: WO 2008/112608

(56) References cited:
- US-A- 3 791 387
- US-A- 5 078 716
- US-A- 5 759 187
- US-A- 6 093 195
- US-A1- 2003 153 909
- US-A1- 2005 119 668
- US-A1- 2005 261 706
- US-A1- 2005 261 706
- US-B1- 6 174 318

## Description

### Field of the Invention

The present invention relates generally to medical devices. More particularly, this invention relates to a medical device for use in endoscopy and polypectomy that performs the dual functions of both a severing or resection of a gastrointestinal (or "GI") polyp from the gastrointestinal tract wall and a trapping and retrieval of the gastrointestinal polyp from the gastrointestinal tract. Alternatively, this invention may also perform as a pure medical retrieval device of polyps, foreign bodies or stones.

### Background of the Invention

Colorectal cancer is the third most common type of cancer in the U.S. In 2007 it is estimated that over 153,000 people will be diagnosed with colorectal cancer and over 52,000 people will die of this disease, making it the number two cancer killer in the United States. Colorectal cancers almost universally must be removed surgically, and a significant portion of patients with colorectal cancer will also require chemotherapy and radiation therapy.

The overwhelming majority of colorectal cancers (about 85-90%) develop from pre-cancerous (adenomatons) colon polyps. Colonoscopy is a medical procedure in which almost all colorectal polyps (big and small) can be found and removed by a technique known as polpectomy.

In a colonoscopy, a colonoscope (a long, flexible tube with a lens and a light source) is inserted into the anus in order to visualize the entire colorectum. The colonoscope contains a long channel through which the doctor can pass various medical devices for both diagnostic and therapeutic purposes. The diameter of this channel usually ranges from 2.8 - 4.2 mm depending on the manufacturer and specific model of colonoscope being used.

Current guidelines recommend that all colorectal polyps be completely removed and sent for a pathologic evaluation to determine if they are pre-cancerous or cancerous. Polyp removal is a 2-stage process. First, the polyp is resected or severed from the colorectal wall. Second, the severed polyp is retrieved from the inside of the colorectum.

Colonoscopy with complete removal of all polyps is the most effective way to reduce the incidence and death rates from colorectal cancer. Reductions in the incident rate of colorectal cancer is reported to be as high as 90% when the techniques of colonoscopy and polypectomy are properly utilized.

A colonoscopy is recommended for healthy people over 50 years of age; healthy people under 50 years of age with a family history of colorectal cancer; people with blood in their stool, a change in their bowel habits, or significant abdominal pain; and people who are iron-deficient which might indicate slow blood loss from the digestive tract. Approximately 80% of adult colonoscopies in the U.S. are performed on patients over 50 years of age.

In 1999, approximately 4.4 million colonoscopies were performed in the U.S. That number is now likely closer to 5 million per year due to increased public awareness (i.e., Katie Couric) and more endoscopists graduating from GI training programs in recent years. According to the CDC, however, about 41.8 million people aged 50 or older in the U.S. have not undergone screening for colorectal cancer as recommended by current guidelines.

Precancerous colorectal polyps are found in about 25-30% of patients over age 50 undergoing routine screening (i.e., no symptoms). Polyp size can vary from 1-2mm all the way to greater than 6 cm. The probability of finding cancer within a colorectal polyp increases with increasing size.

Diminutive colorectal polyps are 1-5 mm in size. They have the lowest risk of being pre-cancerous or cancerous. Presently, resection and retrieval of diminutive colorectal polyps are accomplished using a single medical device, a biopsy forceps, which is removed from the colonoscope with the polyp trapped in the jaws of the forceps.

Small colorectal polyps are 6-9 mm in size. They present a low cancer risk and an intermediate risk of being pre-cancerous. They are usually resected with a snare loop (with or without electric current). Colorectal polyps 8 mm in size or less are usually retrieved via suctioning the specimen through the colonoscope into a specialized collection device. A significant number of these small polyps are lost at some point after entry into the colonoscope and are never retrieved.

Large colorectal polyps are greater than or equal to 1 cm in size. About 20% of colorectal polyps are in this category. The majority of these polyps are at least pre-cancerous. The risk of cancer increases directly with size. Resection is achieved with a snare loop (almost universally with cautery). Due to their size, they are too large to be suctioned into the colonoscope. As such, retrieval is more difficult and almost universally requires a medical device for assistance.

As mentioned above, about 41.8 million people in the U.S. currently go unscreened. Screening rates are likely to rise significantly in the near future as other, highly accurate but less invasive screening tests for colorectal cancer and polyps become commercially viable. The additional large colorectal polyps identified by these tests which will need to be removed by colonoscopy will likely number in the millions.

In addition to colorectal polyps, polyps can also occur in the upper gastrointestinal tract. Although there are fewer polyps in the upper GI tract then in the colorectum, their number is not insignificant. About 50,000 - 100,000 large upper GI polyps are removed annually. A significant percentage of these polyps are precancerous and they can often cause abdominal pain and bleeding. Mechanisms for removal of these polyps are similar to those methods used to remove colorectal polyps.

Currently, there are a number of methods used to retrieve GI polyps, especially large polyps. Six of the more common methods are piecemeal retrieval, suctioning the polyp to the tip of an endoscope, use of the snare as a retriever, use of a grasper device, use of a basket device, and use of retrieval net. Each method has its disadvantages. It is estimated that between 5.7% and 16.5% of resected colorectal polyps are never retrieved. In addition to patient dissatisfaction, this can lead to misdiagnosis as well as the performance of unnecessary surgeries and attendant increases in morbidity and mortality. An additional percentage of polyps which are retrieved require significant increases in time to do so under the current art.

Piecemeal retrieval involves chopping the polyp up into pieces small enough to be suctioned through the endoscope. This occasionally occurs out of necessity with large, flat polyps which cannot be resected in one piece. A pathologist, however, will be unable to determine if resection is complete. Thus, the colonoscopy will often need to be repeated earlier then planned to re-inspect the polypectomy site for residual polyp tissue. Also, part of the specimen (possibly containing early cancer) may not be retrieved.

The suctioning of a polyp to an endoscope tip may work adequately for some rectal polyps. There is, however, a significant risk of the polyp being dropped as suction is inadequate to reliably secure the polyp. A dropped polyp can be lost or (in the case of upper GI tract polyps) can lead to a catastrophic outcome if it is dropped into the trachea.

The use of a snare to retrieve polyp is an attractive option to many endoscopists. It saves time needed to change devices and saves money because there is no added cost in using the same device to retrieve as well as resect the polyp. Snares cost about ten to twenty dollars each. A snare, however, is two dimensional. Thus, while the polyp is easily acquired, it is not secure and can be easily dropped. In addition, the snare can inadvertently bisect the polyp, leading to difficulty in pathologic interpretation as well as the loss of part or all of the polyp.

Graspers usually have three prongs, although four or five pronged graspers are available. Graspers decrease the rate at which specimens are dropped, although some specimens are still dropped because of the grasper's open distal design. Also, the grasper is a second device, different from the snare used for resection, and deploying the grasper adds time to the procedure and increases the chance of losing the specimen during this time interval. Graspers also add costs (about seventy-five dollars) to the procedure.

Document US 2005/119668 A1 discloses a medical device for trapping tissue, polyps, stones and other foreign bodies for retrieval from a body comprising:
a control unit; an elongated tubular element having an inner channel, wherein the tubular element extends from the control unit to a distal end of the tubular element;
two legs forming a snare configuration as a loop at the distal end of the inner channel, wherein the single loop can be at least partially retracted into and extended beyond the distal end of the inner channel via the control unit; the device further comprising additional legs to trap and retrieve the tissue, polyps, stones and other foreign bodies.

When using a basket device, the specimen is secure if it is acquired properly. However, it is harder to acquire very large polyps due to the basket's small size. The basket is also a second device whose deployment adds time to the procedure and risks the specimen being lost. There is also an added cost of over two hundred dollars each for using a basket.

Retrieval nets make it easier to acquire a specimen but the acquisition is less secure because the nets can tear. Also nets can be used to acquire multiple specimens. Again, however, as is the case with basket devices and graspers, the nets are a second device requiring time to deploy them. This creates a risk that a specimen can be lost. Also, the use of baskets increases the cost of a procedure by about seventy-five to eighty-five dollars.

Accordingly, there is a need for an improved polyp removal device for removal of small and large GI polyps.

There is also a need for an improved polyp removal device for removal of small and large GI polyps which makes it easy to acquire and retrieve a specimen, such as a resected polyp.

There is a further need for an improved polyp removal device for removal of small and large GI polyps which decreases the chance that a specimen can be lost.

There is a still further need for an improved polyp removal device for removal of small and large GI polyps which does not require the deployment of a second device which can, among other things, increase the time a procedure takes and increase the possibility that a specimen can be lost.

There is yet another need for an improved polyp removal device for removal of small and large GI polyps which does not increase the cost of a procedure.

### Objects of the invention

It is an object of this invention to provide for an improved polyp removal device for removal of small and large GI polyps.

It is also an object of this invention to provide for an improved polyp removal device for removal of small and large GI polyps which makes it easy to acquire and retrieve a specimen, such as a resected polyp.

It is a further object of this invention to provide for an improved polyp removal device for removal of small and large GI polyps which decreases the chance that a specimen can be lost.

It is a still further object of this invention to provide for an improved polyp removal device for removal of small and large GI polyps which can both resect and retrieve a polyp and as such does not require the deployment of a second device.

It isyet another object of this invention to provide for an improved polyp removal device for removal of small and large GI polyps which does not increase the cost of a procedure.

In addition to the above, it is an object of this invention to provide for a medical basket retrieval device with improved acquisition capabilities which can be used to retrieve other resected polyps, foreign bodies, stones and the like.

These and other objects of the invention are satisfied by the invention described more fully below.

### Summary of the Invention

The present invention relates to a medical device for use in endoscopy and polypectomy as defined in claim 1. The device performs the dual functions of resection and retrieval with decreased possibility that a specimen will be lost or damaged. In the alternative, the present invention can be used to retrieve other polyps, foreign bodies, stones and the like. The present invention functions by use of a novel "Two-Stage Snare-Basket". This and other embodiments of the present invention are discussed more fully below. Preferred embodiments are defined in the dependent claims.

### Description of the Drawings

Fig. 1A shows a top view of the inventive device in a closed position.

Fig. 1B shows a top view of the inventive device in an open position, i.e. with the snare deployed.

Fig. 2A shows a view of the distal end of the inventive device in the open position where the snare is surrounded by one type of sheath and is in a first snare configuration.

Fig. 2B shows a view of the distal end of the inventive device in the open position where the sheath is withdrawn and the device is in a second basket configuration.

Fig. 3A shows a view looking at the distal end of the inventive device, where the wires of the snare are one on top of the other and in an undeployed configuration, i.e. a first snare configuration.

Fig 3B shows a view of the Fig. 3A device which is in its deployed or second basket configuration.

Fig. 3C shows a view similar to that of Fig. 3A, where the snare wires cross over each other to form an X configuration at their distal point, shown here as a cup.

Fig. 3D shows a view of the Fig. 3C in its deployed or second basket configuration.

Fig. 3E shows a view looking at the distal end of the inventive device, where the wires of the snare are configured one on top of the other and in an undeployed configuration, i.e. a first snare configuration.

Fig. 3F shows a view of the Fig. 3E device which is in its deployed or second basket configuration.

Fig. 3G shows a view looking at the distal end of the inventive device, where the wires of the snare are configured one on top of the other and cross over each other to form an X configuration at their distal end, and which is in an undeployed configuration, i.e. a first snare configuration.

Fig. 3H shows a view of the Fig. 3G device which is in its deployed or second basket configuration.

Fig. 4A through Fig. 4E shows one embodiment of the present invention in use to resect and retrieve a polyp.

### Detailed Description of the Invention

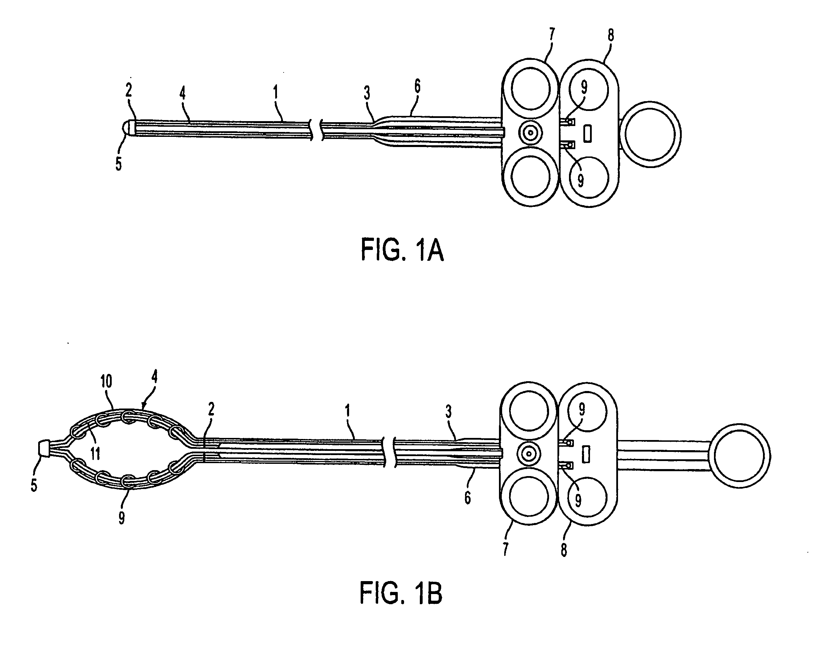

In a preferred embodiment, the present invention, referred to herein as the "Two-Stage Snare-Basket," is a medical device that can be passed through a colonoscope, upper gastrointestinal endoscope, duodenoscope or an enteroscope and operated during a colonoscopy, upper gastrointestinal endoscopy (esophagogastroduodenoscopy), ERCP (endoscopic retrograde cholangio-pancreatography) or enteroscopy procedure, respectively. The Two-Stage Snare-Basket comprises a handle on one end of an elongated tubular element that is connected to and controls a first snare configuration on the other end in the first stage of operation that can be converted into a second basket configuration in the second stage of operation. Initially, the first snare configuration is in a closed position, as shown in Fig. 1A. It can be converted to the open or first snare position, as shown in Fig. 1B, by for example, pushing on a handle (such as (7), (8) of Fig. 1A and Fig. 1B, described in more detail below). Stored energy in the snare will cause it to assume its first snare position. In an alternate embodiment, the snare can spring out of its closed position to its first snare configuration using pre-loaded energy, such as a spring. It should be noted that by whatever means is employed to convert the snare from the closed position to the open position, all of the snare wires, be they full snare wires or half snare wires (discussed more fully below), preferably extend simultaneously.

The first snare configuration in the first stage functions as a resection snare with or without electrocautery to sever the polyp (or portion of an extremely large polyp in a multi-step breakup and removal process) from the GI tract wall and initiates acquisition of the resected polyp (or severed portion of an extremely large polyp). The first snare configuration is easily converted into the second basket configuration for the second stage by control of the handle. The second basket configuration functions in the second stage by control of the handle as a polyp trapping or retrieval element to remove the severed polyp (or severed portion of an extremely large polyp in a multi-step process) from the inside of the GI tract.

In a preferred embodiment, the first snare configuration is comprised of two or three (or more) snares that are juxtaposed on top of one another and/or inside of one another and are held in snare configuration by a stored energy mechanism. Each of the two or three (or more) snares may be comprised of a whole snare (one looped wire each) or two half-snares (two wires that are connected in and/or held by a distal cap) that run from the handle end through the elongated tubular element to a distal cap that permits flexibility of movement of the snare wires between the first snare configuration and the second basket configuration. In this preferred embodiment, the change from the first snare configuration to the second basket configuration is effected via the release of stored mechanical energy at or just proximal to the site of the snare basket itself via a detent mechanism. One stored energy mechanism can be a sheathing element which consists of a wire that holds the snare wires together and runs parallel to the snare wires to the handle end. The sheathing element can be in the form of rings, or alternatively a solid sheath, or spring-loaded hooks. It is understood, however, that while this embodiment and other embodiments of the present invention employ a detent mechanism, the present invention is not limited to the use of detent mechanisms only.

In a preferred embodiment, the first snare configuration is designed such that it can be looped over to surround the polyp in the extended configuration. The first snare configuration can then be tightened by operation of the handle and then at least one of the snares in the first snare configuration is operated as a cauterization tool. The snare with a cautery function will be attached to a source of electricity through an electrocautery snare plug or other mechanism located within the device handle. Following cauterization and resection of the polyp, the snare configuration can then be held in position surrounding the resected polyp (or severed portion of an extremely large polyp).

The first snare configuration of the preferred embodiment is easily converted by control of the handle to the second basket configuration by retracting or releasing the sheathing element that holds the various snare wires together during polyp retrieval. When the sheathing element is retracted, the two snares spring or rotate apart and assume the second basket configuration.

Following conversion of the first snare configuration into the second basket configuration, the second basket configuration can be tightened around the resected polyp (or severed portion of an extremely large polyp) by control of the handle to securely trap the resected polyp (or severed portion of an extremely large polyp) for retrieval.

In various embodiments of the invention, the size and shape of the snare and basket may vary, angulation of wires in the basket configuration may vary, and the length and the diameter of the medical device and the elongated tubular element may also vary.

In various embodiments of the invention, the number of snares within the first snare configuration (and accordingly the number of wires in the corresponding second basket configuration) may vary and whether the snares consist of whole snares or half-snares may vary. The arrangements and connectivity of the half-snares and whole snares may also vary. In addition, whether one or more of the snares or the sheathing element functions as the cauterization tool may vary. The methods or mechanism of conversion from the first snare configuration to the second basket configuration may also vary.

In various embodiments of the invention, the sheathing element may vary. The sheathing element can be in the form of rings, spring-loaded hooks or a solid sheath. In other embodiments of the invention, there may not be a sheathing element, but instead, the device may contain a rotational or flip lever detent mechanism which does not utilize the release of stored energy to effect the conversion from the first snare configuration to the second basket configuration.

In various embodiments of the invention, the handle that controls the first snare configuration and second basket configuration and conversion between the two configurations may vary. The handle may be comprised of a single handle with two or more levers and a locking device or may be a combination of any number of handles, any number of levers, and any number of locking devices. A locking device may not even be necessary in various embodiments. The location and design of the electrocautery plug may also vary.

This invention has the potential to save time and lower costs in that one single device will perform both resection and retrieval of polyps rather than two separate devices. For instance, in removal of large colorectal polyps in particular, resection or severing is usually accomplished by one device and trapping and retrieval is usually accomplished by a second device.

This invention may also simplify the retrieval of large polyps, which can often be more difficult to trap and retrieve due to polyp size and shape, and if extremely large, may require a multi-step breakup and removal. In the first snare configuration, for instance, the open two-dimensional design at the initial stage of acquisition provides a larger space than the narrow space between the wires in prior basket devices. This invention is also capable of repeated uses to resect and retrieve multiple polyps (or multiple portions of an extremely large polyp) from the same patient during a single colonoscopy procedure. This invention may also simplify the removal of small polyps by achieving a method for complete pull-through-the-endoscope removal of small polyps analogous to how biopsy forceps provide a method for complete pull-through-the-endoscope removal of diminutive polyps.

This invention may also function as a pure retrieval device for already severed polyps (or severed portions of extremely large polyps), foreign bodies and stones in the gastrointestinal tract, or in other organs or body cavities. For use as a pure retrieval device, the invention would not require electrocautery function and the corresponding electrocautery elements of the invention for resection of a polyp would not be required.

In fact, the present invention has numerous applications, and represents a major improvement in specimen acquisition for basket retrieval technology. For polyp removal, it can be used in the gastrointestinal tract, such as the colorectal, small intestinal, gastric and esophageal areas. For gynecological polyps, it can be used in the uterer, cervix and vagina. The present invention can also be used to remove polyps in the genitourinary tract, such as the bladder and urethra. The present invention is also useful in removing ear, nose and throat polyps, such as nasal, palatal, oral and vocal cord polyps. In addition, the present invention can be used to remove polyps in the peritoneal cavity.

The present invention can also be used for stone removal. This includes stones in the gastrointestinal tract, such as the biliary tree (intra-hepatic and extra-hepatic), pancreas and gallbladder. It can also be used in the genitourinary tract to remove stones in the kidney, bladder and ureter.

Furthermore, the present invention is useful in foreign body retrieval. For example, the present invention can remove foreign bodies in the gastrointestinal tract, such as colorectal, gastric, esophageal and small intestinal foreign bodies. It can remove foreign bodies from the genitourinary tract, such as from the urethra and bladder. It can be used for gynecological foreign body removal, such as in the vagina, cervix and uterus. It can be used to remove intra-abdominal or intra-peritoneal foreign bodies, such as during laparoscopic gallbladder procedures. Furthermore, it can be used to remove foreign bodies from the thoracic cavity, such as debris, medical instruments and resection specimens.

Turning now to the drawings, Fig. 1A shows the inventive device in the closed position, i.e. where the snare (4) is not deployed. The device comprises an elongated tubular element or a tube (1) with a distal end (2) and a proximal end (3). The tube (1) is preferably flexible and can be made of, for example, catheter based plastic. The tube (1) preferably has an outside diameter from about 2.8 mm to about 4.2 mm, more preferably about 2.8 mm to about 3.7 mm.

The tube (1) contains a snare (4) at its distal end (2), which terminates in a cap (5).

The proximal end (3) of the tube (1) is connected to a control unit or a grip (6). The grip (6) is preferably less flexible then the tube (1). The grip (6) can be made of, for example, plastic or metal.

A first slidable handle (7) and a second slidable handle (8) are slidably disposed on the grip (6). There is an optional connection means (not shown) which connects the first slidable handle (7) to the second slidable handle (8). That way, the two handles (7, 8) can move in unison. The connecting means is disengageable so that the slidable handles (7, 8) can be moved independently of each other.

Fig 1B shows the device with the snare (4) extended or deployed, i.e. in a first snare configuration. Part of the snare (4) is a sheath (9) which surrounds a first snare wire (10) and a second snare wire (11). The snare wires (10, 11) are held together by the sheath (9). Also, the sheath (9) extends through the tube (1), into the grip (6) and attaches to the second slidable handle (8). The snare wires (10, 11) also pass through the tube (1) and into the grip (6) to attach to the first slidable handle (7) via a means described more fully below.

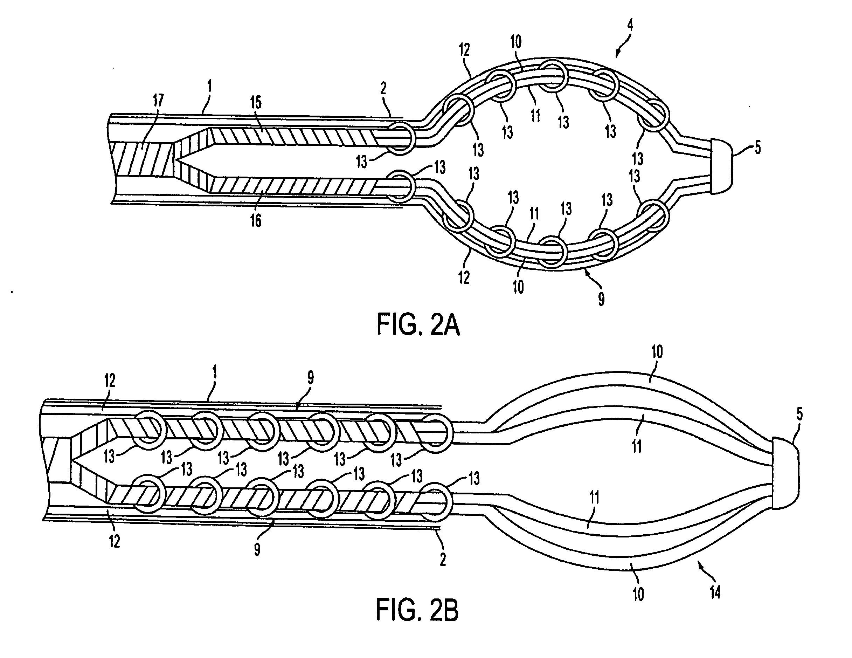

Fig. 2A and Fig. 2B show the snare (4) in more detail. Fig. 2A shows the snare (4) in a deployed or first snare configuration. It is surrounded by the sheath (9) which keeps the first snare wire (10) and the second snare wire (11) together. In the particular embodiment shown in Fig. 2A, the sheath (9) comprises a sheath backing (12) and a plurality of sheath rings (13). In this configuration, it is the sheath backing (12) which passes through the tube (1) into the grip (6) and connects to the second slidable handle (8), the attachment shown as (9) in Fig. 1A and Fig. 1B.

As shown in Fig. 2A and Fig. 2B, as well as Fig. 1B, the snare wires (10, 11) terminate in a cap (5). It is understood, however, that instead of a cap (5), one or both of the snare wires (10, 11) could form a continuous loop (not shown). The wires (10, 11) of the snare basket device (4, see also 14) at about, just proximal to, or at their distal ends can fuse into or attach to a single wire or braid into each other at about, just proximal to, or at, the most distal aspect of the snare basket device (4, see also 14), thereby forming the most distal aspect of the snare basket device. The distal end of the snare basket device (4, see also 14) is not left open, but rather the snare wires (10, 11) will connect to each other as described above either directly (braiding, fusion, etc.) or indirectly (via a cap (5) or intervening wire or wires, etc.).

As discussed above, it is understood that the sheath (9) can have various configurations. The configuration shown in, for example, Fig. 2A shows the sheath made of metal sheath rings (13) supported by a wire sheath backing (12). Alternatively, the sheath can be solid, such as a flexible metal member or flexible plastic or rubber member, or the rings can be partial rings, i.e. not fully closed, or spring loaded hooks or clips. Alternatively, pincers oriented from distal to proximal or proximal to distal can be used to hold - the snare wires (10, 11) together. The pincers can be released, for example by a spring loaded means, or simply withdrawn into the tube (1) to allow the formation of the second basket configuration (14) as shown in Fig. 2B. Two to four pincers arraigned as up-down or left-right pairs may be present on each side of the snare (4).

It should also be noted that although Fig. 2B shows the sheath (9) being withdrawn proximally into the tube (1), in an alternative embodiment the sheath (9) can be pushed to the distal end of the snare (4).

The snare (4) as shown in Fig. 1B and Fig. 2A, or the second basket configuration (14) of Fig. 2B, is made of two complete snare wires (10, 11), although the invention is not so limited. For example, there can be three, four or more snare wires used in the present invention. Likewise, one or more (but not all) of the snare wires can be half wires. A half wire will also be connected to the cap (5) or otherwise connected to the other snare wires (10 or 11).

The snare wires (10, 11), can be flat wire, braided wire or shaped memory wire, or any suitable material which can function as a first snare configuration (4) and a second basket configuration (14).

Preferably, the snare wires (10, 11) are made of stainless steel, nickel titanium alloy (Nitinol) or titanium.

The snare wires (10, 11) as shown in Fig. 2A are adjacent to each other in the first snare configuration (4). Alternatively, from a top view, they can be juxtaposed to be one on top of the other. Within the tube (1), the snare wires (10, 11) first braid with each other to form first braids (15, 16) and those first braids (15, 16) are in turn braided together to form a second braid (17). This second braid (17) then passes through the tube (1) and into the grip (6) to attach to, in the embodiment shown in Fig. 1A and Fig. 1B, the first slidable handle (7).

It is understood that the braiding is not necessary to practice the present invention. For example, the snare wires (10, 11) can simply pass through the tube (1) and into the grip (6) to connect with the first slidable handle (7). An alternative embodiment can use different materials. For example, the actual portion of the snare wires (10, 11) which form the first snare configuration (4) and the second basket configuration (14) can be made of stainless steel, Nitinal or titanium. Then, in place of the second braid (17) or even the first braids (15, 16) the same or different material can be used as a single piece. Thus, this single piece can be stainless steel, titanium, nitinal, or a different metal, or even a non-metallic substance, such as plastic. The first braids (15, 16) or even the snare wires (10, 11) themselves can be attached to this single piece by conventional means, such as welding, soldering, clamps, glue and the like.

The formation of the second basket configuration (14) can occur in a number of ways. In one preferred embodiment, the snare wires (10, 11) are made of a shaped memory wire or otherwise configured so that in their resting or unconstrained state, they would assume the second basket configuration (14). They are held in their first snare configuration (4) by the constraining action of a detent mechanism utilizing stored energy. This detent mechanism might utilize, among other mechanisms, rings, hooks, pincers or sheathing. This detent mechanism might be effected by a spring or the manipulation of the handle in order to release the stored energy to effect the change in configurations. The spring can be located in the tube (1) and can be activated when desired, such as by pushing a button which will release the spring. In another embodiment, a torque can be applied to the snare wires (10, 11) to rotate them from the first snare configuration (4) to their second basket configuration (14). Torque can be applied by any conventional means. For example, the first slidable handle (7) may also be rotatable, the rotation providing torque. There may also be a torque means, such as a wheel or knob, which can be turned to provide torque. This torque means can be present, for example, somewhere on the tube (1) or grip (6), or can even be placed on the first slidable handle (7).

In an alternate embodiment (not shown) a support means can be provided at the distal end (2) of the tube (1). This support means prevents deformation, fraying or tearing at the distal end (2) of the tube (1). It can be in the shape of a ring or a cap and is made of any suitable material, such as plastic.

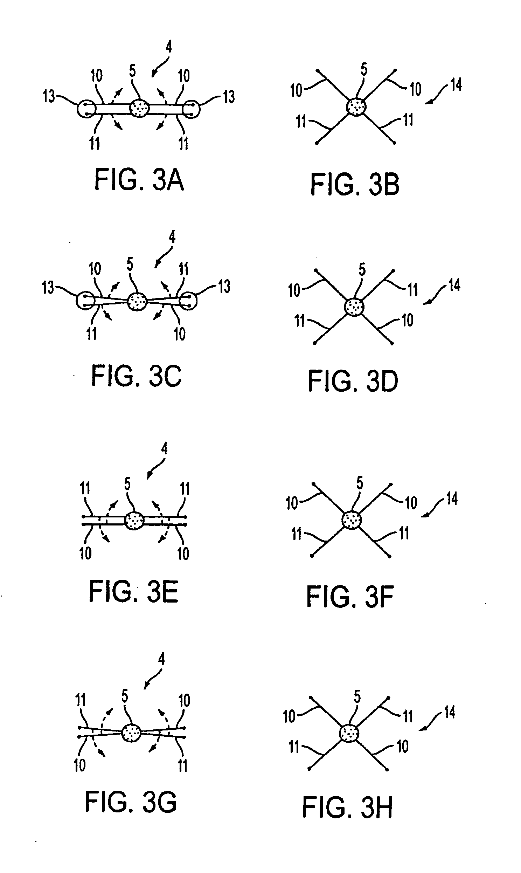

The configuration of the snare wires (10, 11) relative to each other can take several different configurations. These are shown in more detail in Fig. 3A through Fig. 3H, which shows just the snare wires (10, 11) and the cap (5) from a head-on perspective.

Fig. 3A is similar to the configuration of Fig. 2A. In both, one (or first) snare wire (10) is positioned outside the other (or second) snare wire (11) when in the first snare configuration (4). Rings (13) are also shown. When in the second basket configuration (14) of Fig. 2B and Fig. 3B, first snare wire (10) positions up and second snare wire (11) positions down, thus forming a basket. In Fig. 2B, first snare wire (10) would be positioned above the plane of the page and second snare wire (11) would be positioned below the plane of the page.

In an alternative embodiment, the snare wires (10, 11) of Fig. 3C are configured in a manner similar to Fig. 3A except that they cross over each other to form an X configuration at the cap (15). The snare wires (10, 11) move, as shown by the dotted lines in Fig. 3C, to form the second basket configuration (14) of Fig. 3D.

Fig. 3E shows an embodiment where the first snare wire (10) is under the second snare wire (11) in the first snare configuration (4). The top snare wire (11) has a downward force and the bottom snare wire (10) has an upward force. This holds the snare wires (10, 11) together in the first snare configuration (4) even without, for example, the rings (13) (not shown). When the snare wires (10, 11) are moved, so that they are no longer in contact with each other, the bottom snare wire (10) positions up and the lower snare (11) positions down form the second basket configuration (14) of Fig. 3F.

Fig. 3G shows the first snare wire (10) and the second snare wire (11) cross over each other at the cap (5) to form an X configuration when in the first snare configuration (4). In a manner similar to that of Fig. 3E, the upper snare wires (11 to the left of the cap (5), 10 to the right of cap (5)) have a downward force and the lower snare wires (10 to the left, 11 to the right) have an upward force. To achieve the second basket configuration (14) of Fig. 3H, the top portion of each snare wire (10, 11) positions up and the bottom portion of each snare wire (10, 11) position down, as shown in Fig. 3H. This can be done by, for example, moving the snare wires (10, 11) relative to each other, in a manner similar to that described for Fig. 3E and Fig. 3F, above.

Of course, as described above, the cap (5) need not be present. Each snare wire (10, 11) can simply be a loop and be attached to each other at their distal ends.

As discussed above, and especially for polyp removal, the present invention can cauterize the polyp to resect it. A conventional source of electricity can be provided. In one embodiment, one or more of the snare wires (10, 11) performs the cautery function. In an alternate embodiment, this configuration may not be optimal because some of the cauterized sample may adhere to the snare wires (10, 11) and prevent or inhibit their deployment in the second basket configuration (14). To avoid this, the current for cauterization can pass through the sheath (9), which can be made, of a conducting material or contain a conducting material, such as a wire. To complete the circuit, the distal ends of the sheath (9) should be in contact or connected by, e.g., a wire or cap (5). In an alternative embodiment, current can pass through both the sheath (9) and one or more of the sheath wires (10, 11). Since only the sheath (9) will contact the sample for cauterization, little or no portion of the cauterized sample will contact any of the sheath wires (10, 11).

It should be noted that in an embodiment of the present invention a sheath is not used. For example, the snare wires (10, 11) may not have a shaped memory and when deployed in a first snare position (4) they will stay in close proximity to each other. Transition to the second basket configuration (14) can be effected by rotating one or both snare wires (10, 11) such as, for example, by applying torque, as previously described or through a detent mechanism utilizing for example flip levers or dials as previously described. In another embodiment, the snare wires (10, 11) are configured one on top of the other. The top snare wire (11) is made of shaped memory or is otherwise so configured to push down and the bottom snare wire (10) is configured to push up. These opposing forces hold the snare wires (10, 11) together in a first snare configuration (4). In alternate embodiments, where these snare wires (10, 11) contact each other can be a flat surface, a channel can be provided or a tongue and groove type system can be employed to more securely hold them in place. To convert to the second basket configuration (14) the snare wires (10, 11) can be simply moved relative to each other, i.e. front, back or side to side. Once the snare wires (10, 11) are no longer in contact with each other, they will snap into their second basket configuration (14). This is shown in, for example, Fig. 3E and Fig. 3F.

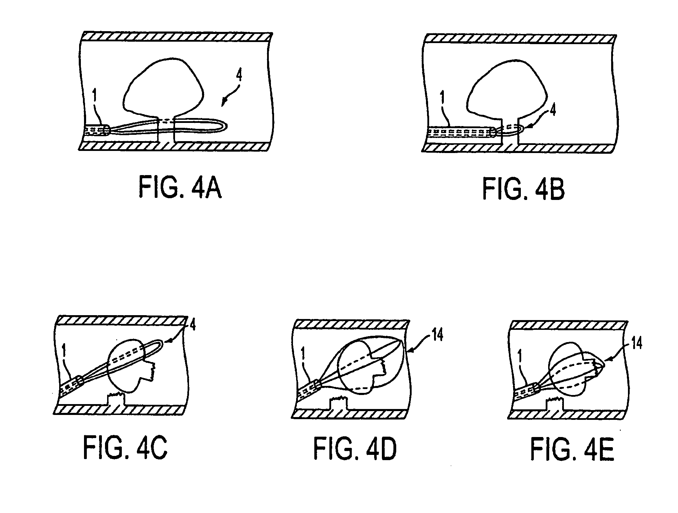

Fig. 4 shows one embodiment of the present invention used to resect and remove a colorectal polyp. As shown in Fig. 4A, once a polyp is located the first snare configuration (4) is used to surround the polyp. The snare (4) is then tightened and the polyp is cauterized, as shown in Fig. 4B. The first snare configuration (4) is then enlarged, if necessary, to surround the cauterized polyp, as shown in Fig. 4C. In Fig. 4D, the first snare configuration (14) is converted into the second basket configuration (14) by the means disclosed above. As shown in Fig. 4E, the second basket configuration (14) has been tightened around the resected polyp completing the highly secure acquisition process. The polyp is then removed, either by withdrawing the entire snare basket device out of the therapeutic channel of the endoscope (small polyp) or by withdrawing both the entire snare basket device and endoscope from the GI tract cavity in tandem (large polyp).

In another embodiment of the present invention, the sheath (9) can be used to control how many snare wires (10, 11) are released to form the second basket configuration (14). This may be necessary, for example, where space is limited. Referring to Fig. 2A and Fig. 2B, instead of withdrawing the entire sheath (9), only the left half or the right half is withdrawn. In effect, this forms a second basket configuration (14) with three wires instead of four wires.

As mentioned above, an endoscope includes a lens with a light source, as well as a means for viewing the acquired images. In addition, endoscopes include a therapeutic channel through which medical device accessories can be passed and whose diameter is usually between 2.8 mm and 4.2 mm. While not shown herein, it is understood that the present invention is compatible with these features.

In view of the above, and further in view of the drawings, especially Fig. 1A, Fig. 1B and Fig. 2B, the functioning of one embodiment of the present invention is apparent. An endoscope is inserted into the GI tract via the mouth, anus or a previously created surgical ostomy and is advanced to the desired segment of the GI tract via the operator. If a polyp is found, the inventive device is inserted into the therapeutic channel of the endoscope and is advanced through that therapeutic channel until its distal end extends out of the distal end of the therapeutic channel of the endoscope and into the GI lumen which contains the polyp. The first slidable handle (7) and the second slidable handle (8) are slid together in a distal direction, thus deploying the snare (4). The snare (4) is positioned around the polyp and pulled tight by pulling the first slidable handle (7) and second slidable handle (8) together in a proximal direction. Once properly positioned, an electric current is passed through the snare (4) to cauterize the polyp. The snare (4) can be enlarged again, if necessary, to surround the polyp by pushing the first slidable handle (7) and the second slidable handle (8) together in a distal direction. This also helps to align the polyp within the snare initiating polyp acquisition. Once the polyp is aligned, the second slidable handle (8) is pulled in a proximal direction, but the first slidable handle (7) is not moved. This pulls the sheath (9) down and off the snare wires (10, 11). Once the sheath (9) is removed from the snare wires (10, 11), they convert into the second basket configuration (14) because the sheath (9) is no longer holding them in place. This furthers the highly effective process of polyp acquisition. The second basket configuration (14) can then be closed as much as necessary in order to tighten the second basket configuration around the polyp to secure and complete acquisition of the polyp by pulling the first slidable handle (7) proximally until resistance to further sliding is felt indicating that the polyp is securely acquired within the second basket configuration (14). The second basket configuration (14) with the polyp secured can then be withdrawn through the endoscope (small polyp) or the entire endoscope assembly including the second basket configuration (14) with the polyp secured can be withdrawn (large polyp). Once removed from the body, the polyp is discharged from the second basket configuration (14). The sheathing (9) can be re-advanced over the snare wires (10, 11) either manually or by sliding the first slidable handle (7) and second slidable handle (8) together to reform the first snare configuration (4). The first snare configuration (4) is then closed, as shown in for example Fig. 1A. The device is then ready for continued use in the patient.

The disclosed embodiments are illustrative of the various ways in which the present invention may be practiced. Other embodiments can be implemented by those skilled in the art without departing from the scope of the present claims.

## Claims

1. A medical device for trapping tissue, polyps, stones and other foreign bodies for retrieval from a body comprising:
a control unit (6);
an elongated tubular element (1) having an inner channel,
wherein the tubular element extends from the control unit (6) to a distal end of the tubular element;
at least two snare wires (10, 11) forming a first snare configuration, extending from the control unit through the inner channel of the tubular element, and juxtaposed and held in a single loop at the distal end of the inner channel, wherein the single loop can be at least partially retracted into and extended beyond the distal end of the inner channel via the control unit (6); and the
at least two snare wires (10, 11) forming a second basket configuration by converting the first snare configuration via the control unit (6), wherein the second basket configuration is operative to trap and retrieve the tissue, polyps, stones and other foreign bodies from a body via the control unit.

2. A medical device according to claim 1, wherein the snare wires that are juxtaposed and held in the single loop in the first snare configuration are held in the single loop by a sheathing element, wherein the sheathing element (9), via the control unit, can be retracted to convert the first snare configuration into the second basket configuration and extended to convert the second basket configuration into the first snare configuration.

3. A medical device according to claim 1, wherein the snare wires that are juxtaposed and held in the single loop in the first snare configuration are held in the single loop by at least one lever that, via the control unit, can be released to convert the first snare configuration into the second basket configuration and engaged to convert the second basket configuration into the first snare configuration.

4. A medical device according to claim 1 for resecting a gastrointestinal polyp from a colorectal wall and trapping the resected polyp for retrieval from a colorectum,
wherein the tubular element extends from the control unit to a distal end of the tubular element through the inner channel; and
wherein the single loop can be operated as a cauterization tool to resect a polyp from a colorectal wall by connection to an electrical source.

5. A medical device according to claim 1 or 4, wherein the second basket configuration can be converted into the first snare configuration via the control unit.

6. A medical device according to claim 4, wherein at least one snare wire is the cauterization tool through connection to an electrical source.

7. A medical device according to claim 1 or 4, wherein at least one snare wire contains one continuous looped wire.

8. A medical device according to claim 1 or 4, wherein at least one snare wire contains at least two connected wires and at least one mechanism for connecting the wires.

9. A medical device according to claim 4, wherein the snare wires that are juxtaposed and held in the single loop in the first snare configuration are held in the single loop by a sheathing element (9), wherein the sheathing element via the control unit can be retracted to convert the first snare configuration into the second basket configuration and extended to convert the second basket configuration into the first snare configuration.

10. A medical device according to claim 2 or 9, wherein the sheathing element is in the form of rings (13), in the form of a solid sheath or in the form of spring-loaded hooks.

11. A medical device according to claim 9, wherein the sheathing element is the cauterization tool through connection to an electrical source.

12. A medical device according to claim 4, wherein the snare wires that are juxtaposed and held in the single loop in the first snare configuration are held in the single loop by a lever that via the control unit can be released to convert the first snare configuration into the second basket configuration and engaged to convert the second basket configuration into the first snare configuration.

13. A medical device according to claim 3 or 12 containing at least one rotational lever or at least one flip lever.

14. A medical device according to claim 1 or 4, wherein the control unit includes at least one handle and at least one sliding component.

15. A medical device according to claim 1 or 4, wherein the control unit includes at least one handle and at least one locking device.

## Patentansprüche

1. Medizinische Vorrichtung zum Einfangen von Gewebe, Polypen, Steinen und anderen Fremdkörpern, die aus einem Körper geborgen werden, umfassend:
eine Steuereinheit (6);
ein längliches, rohrförmiges Element (1) mit einem Innenkanal, wobei sich das rohrförmige Element von der Steuereinheit (6) zu einem distalen Ende des rohrförmigen Elements erstreckt;
mindestens zwei Drahtschlingen (10, 11), die eine erste Schlingenkonfiguration bilden, die sich von der Steuereinheit durch den Innenkanal des rohrförmigen Elements erstreckt, und nebeneinander liegen und am distalen Ende des Innenkanals in einer Einzelschlinge gehalten werden, wobei die Einzelschlinge über die Steuereinheit (6) zumindest teilweise in das distale Ende des Innenkanals zurückgezogen und über dieses hinaus ausgerückt werden kann; und
wobei die mindestens zwei Drahtschlingen (10, 11) eine zweite Korbkonfiguration durch Umwandeln der ersten Schlingenkonfiguration durch die Steuereinheit (6) bilden, wobei die zweite Korbkonfiguration betriebsfähig ist, um das Gewebe, die Polypen, die Steine und anderen Fremdkörper aus einem Körper über die Steuereinheit einzufangen und zu bergen.

2. Medizinische Vorrichtung nach Anspruch 1, wobei die Drahtschlingen, die nebeneinander liegen und in der ersten Schlingenkonfiguration in einer Einzelschlinge gehalten werden, von einem Ummantelungselement in der Einzelschlinge gehalten werden, wobei das Ummantelungselement (9) über die Steuereinheit zurückgezogen werden kann, um die erste Schlingenkonfiguration in die zweite Korbkonfiguration umzuwandeln, und ausgerückt werden kann, um die zweite Korbkonfiguration in die erste Schlingenkonfiguration umzuwandeln.

3. Medizinische Vorrichtung nach Anspruch 1, wobei die Drahtschlingen, die nebeneinander liegen und in der ersten Schlingenkonfiguration in einer Einzelschlinge gehalten werden, von mindestens einem Hebel in der Einzelschlinge gehalten werden, der über die Steuereinheit gelöst werden kann, um die erste Schlingenkonfiguration in die zweite Korbkonfiguration umzuwandeln, und in Eingriff gebracht werden kann, um die zweite Korbkonfiguration in die erste Schlingenkonfiguration umzuwandeln.

4. Medizinische Vorrichtung nach Anspruch 1 für die Entfernung eines Magendarmpolypen aus einer kolorektalen Wand und zum Einfangen des entfernten Polypen, um diesen aus einem Kolorektum zu bergen,
wobei sich das rohrförmige Element von der Steuereinheit durch den Innenkanal zu einem distalen Ende des rohrförmigen Elements erstreckt; und
wobei die Einzelschlinge durch Anschließen an eine Stromquelle als Verätzungswerkzeug verwendet werden kann, um einen Polypen aus einer kolorektalen Wand zu entfernen.

5. Medizinische Vorrichtung nach Anspruch 1 oder 4, wobei die zweite Korbkonfiguration über die Steuereinheit in die erste Schlingenkonfiguration umgewandelt werden kann.

6. Medizinische Vorrichtung nach Anspruch 4, wobei mindestens eine Drahtschlinge das Verätzungswerkzeug durch Anschluss an eine Stromquelle ist.

7. Medizinische Vorrichtung nach Anspruch 1 oder 4, wobei mindestens eine Drahtschlinge einen kontinuierlich geschlungenen Draht enthält.

8. Medizinische Vorrichtung nach Anspruch 1 oder 4, wobei mindestens eine Drahtschlinge mindestens zwei verbundene Drähte und mindestens einen Mechanismus zum Verbinden der Drähte enthält.

9. Medizinische Vorrichtung nach Anspruch 4, wobei die Drahtschlingen, die nebeneinander liegen und in der Einzelschlinge in der ersten Schlingenkonfiguration gehalten werden, von einem Ummantelungselement (9) in der Einzelschlinge gehalten werden, wobei das Ummantelungselement über die Steuereinheit zurückgezogen werden kann, um die erste Schlingenkonfiguration in die zweite Korbkonfiguration umzuwandeln, und ausgerückt werden kann, um die zweite Korbkonfiguration in die erste Schlingenkonfiguration umzuwandeln.

10. Medizinische Vorrichtung nach Anspruch 2 oder 9, wobei das Ummantelungselement die Form von Ringen (13), die Form einer massiven Ummantelung oder die Form von federbelasteten Haken aufweist.

11. Medizinische Vorrichtung nach Anspruch 9, wobei das Ummantelungselement das Verätzungswerkzeug durch Anschluss an eine Stromquelle ist.

12. Medizinische Vorrichtung nach Anspruch 4, wobei die Drahtschlingen, die nebeneinander liegen und in der Einzelschlinge in der ersten Schlingenkonfiguration gehalten werden, von einem Hebel in der Einzelschlinge gehalten werden, der über die Steuereinheit gelöst werden kann, um die erste Schlingenkonfiguration in die zweite Korbkonfiguration umzuwandeln, und in Eingriff gebracht werden kann, um die zweite Korbkonfiguration in die erste Schlingenkonfiguration umzuwandeln.

13. Medizinische Vorrichtung nach Anspruch 3 oder 12, die mindestens einen Drehhebel oder mindestens einen Kipphebel enthält.

14. Medizinische Vorrichtung nach Anspruch 1 oder 4, wobei die Steuereinheit mindestens einen Griff und mindestens eine Gleitkomponente enthält.

15. Medizinische Vorrichtung nach Anspruch 1 oder 4, wobei die Steuereinheit mindestens einen Griff und mindestens eine Verriegelungsvorrichtung enthält.

## Revendications

1. Dispositif médical pour piéger des tissus, des polypes, des pierres et autres corps étrangers à retirer d'un corps comprenant :
une commande (6)
un élément tubulaire allongé (1) doté d'un canal intérieur, dans lequel l'élément tubulaire s'étend à partir de la commande (6) à une extrémité distale de l'élément tubulaire ;
au moins deux fils à collet (10, 11) formant une première configuration pour piège, s'étendant à partir de la commande à travers le canal interne de l'élément tubulaire, et juxtaposés et maintenus en une seule boucle à l'extrémité distale du canal interne, dans lequel la seule boucle peut être au moins partiellement se rétracter et s'étendre au-delà de l'extrémité distale du canal interne par l'intermédiaire de la commande (6) ; et l'
au moins deux fils à collet (10, 11) formant une seconde configuration en panier en convertissant la première configuration pour piège par l'intermédiaire de la commande (6), dans lequel la deuxième configuration en panier fonctionne pour piéger et retirer des tissus, des polypes, des pierres et autres corps étrangers d'un corps par l'intermédiaire de la commande.

2. Dispositif médical selon la revendication 1, dans lequel les fils à collet qui sont juxtaposés et maintenus dans la boucle unique dans la première configuration pour piège sont maintenus dans la boucle unique par un élément de revêtement, dans lequel l'élément de revêtement (9) peut être rétracté grâce à la commande pour passer de la première configuration pour piège à la deuxième configuration en panier et étendu pour passer de la seconde configuration en panier à la première configuration pour piège.

3. Dispositif médical selon la revendication 1, dans lequel les fils à collet qui sont juxtaposés et maintenus dans la boucle unique dans la première configuration pour piège sont maintenus dans la boucle unique par au moins un levier qui, par l'intermédiaire de la commande, peut être desserré pour passer de la première configuration pour piège à la deuxième configuration en panier et serré pour passer de la seconde configuration en panier à la première configuration pour piège.

4. Dispositif médical selon la revendication 1 pour enlever par résection un polype gastro-intestinal d'une paroi colorectale et piéger le polype réséqué afin de le retirer d'un rectum,
dans lequel l'élément tubulaire s'étend de la commande à une extrémité distale de l'élément tubulaire à travers le canal interne, et
dans lequel la boucle unique peut être utilisée comme un outil de cautérisation pour réséquer un polype colorectal à partir d'une paroi par connexion à une source électrique.

5. Dispositif médical selon la revendication 1 ou 4, dans lequel la deuxième configuration en panier peut être convertie en la première configuration pour piège par l'intermédiaire de la commande.

6. Dispositif médical selon la revendication 4, dans lequel au moins un fil à collet est l'outil de cautérisation après la connexion à une source électrique.

7. Dispositif médical selon la revendication 1 ou 4, dans lequel au moins un fil à collet contient un fil en forme de boucle continue.

8. Dispositif médical selon la revendication 1 ou 4, dans lequel au moins un fil à collet comprend au moins deux fils connectés et au moins un mécanisme pour le raccordement des fils.

9. Dispositif médical selon la revendication 4, dans lequel les fils à collet qui sont juxtaposés et maintenus dans la boucle unique dans la première configuration pour piège sont maintenus dans la boucle unique par un élément de revêtement (9), dans lequel l'élément de revêtement peut être rétracté grâce à la commande pour passer de la première configuration pour piège à la deuxième configuration en panier et étendu pour passer de la seconde configuration en panier à la première configuration en piège.

10. Dispositif médical selon la revendication 2 ou 9, dans lequel l'élément de revêtement se présente sous forme d'anneaux (13) ; sous forme d'une gaine solide ou sous forme de crochets à ressort.

11. Dispositif médical selon la revendication 9, dans lequel l'élément de revêtement est l'outil de cautérisation après la connexion à une source électrique.

12. Dispositif médical selon la revendication 4, dans lequel les fils à collet qui sont juxtaposés et maintenus dans la boucle unique dans la première configuration pour piège sont maintenus dans la boucle unique par un levier qui, grâce à la commande, peut être desserré pour passer de la première configuration pour piège à la seconde configuration en panier et serré pour passer de la seconde configuration en panier à la première configuration pour piège.

13. Dispositif médical selon la revendication 3 ou 12, contenant au moins un levier de rotation ou au moins un levier de bascule.

14. Dispositif médical selon la revendication 1 ou 4, dans lequel la commande comprend au moins une poignée et au moins un élément coulissant.

15. Dispositif médical selon la revendication 1 ou 4, dans lequel la commande comprend au moins une poignée et au moins un dispositif de verrouillage.
